(19) Europäisches Patentamt
European Patent Office
Office européen des brevets

(11) **EP 4 032 475 A1**

(12) **EUROPEAN PATENT APPLICATION**
published in accordance with Art. 153(4) EPC

(43) Date of publication:
**27.07.2022 Bulletin 2022/30**

(21) Application number: **20887306.7**

(22) Date of filing: **19.10.2020**

(51) International Patent Classification (IPC):
***A61B 6/00*** *(2006.01)*

(52) Cooperative Patent Classification (CPC):
**A61B 6/00**

(86) International application number:
**PCT/JP2020/039232**

(87) International publication number:
**WO 2021/095447 (20.05.2021 Gazette 2021/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.11.2019 JP 2019205686**

(71) Applicant: **Canon Kabushiki Kaisha
Ohta-ku
Tokyo 146-8501 (JP)**

(72) Inventor: **NODA, Takeshi
Tokyo 146-8501 (JP)**

(74) Representative: **WESER & Kollegen
Patentanwälte PartmbB
Radeckestraße 43
81245 München (DE)**

(54) **IMAGE PROCESSING DEVICE, RADIOGRAPHY DEVICE, IMAGE PROCESSING METHOD, AND PROGRAM**

(57) An image processing apparatus comprises a region specifying unit configured to specify a region composed of one substance from radiation images corresponding to a plurality of energies and obtained by irradiating an object with radiation, a pixel value estimation unit configured to obtain an estimated pixel value of the radiation image based on a thickness or density of a substance in the region, and a scattered ray estimation unit configured to estimate a scattered ray included in the radiation image from a difference between a pixel value of the radiation image and the estimated pixel value.

**F I G. 1**

EP 4 032 475 A1

**Description**

TECHNICAL FIELD

The present invention relates to an image processing apparatus, a radiation imaging apparatus, an image processing method, and a program.

BACKGROUND ART

A radiation imaging apparatus using a flat panel detector (to be abbreviated as "FPD" hereinafter) is widely used as an imaging apparatus used for medical image diagnosis using radiation. The FPD can perform digital image processing on a captured image. Accordingly, various applications have been developed and practiced.
PTL 1 discloses, as a method of obtaining an image by an energy subtraction method, an arrangement that is provided with a scattered ray removal structure between a front detector and a rear detector, which are stacked on each other, to obtain, from the rear detector, a radiation image from which scattered rays are removed.

CITATION LIST

PATENT LITERATURE

PTL 1: Japanese Patent No. 3476644

SUMMARY OF INVENTION

TECHNICAL PROBLEM

When a radiation image includes scattered rays, the contrast of the radiation image decreases. This can cause a reduction in diagnostic performance. In energy subtraction, the substance decomposition performance with respect to bones, soft tissues, and the like decreases, resulting in difficulty in generating an image with a target substance being separated.
According to the method disclosed in PTL 1, a rear detector needs to have a two layer structure composed of a high-energy detector (FPD) and a low-energy detector (FPD). Accordingly, this method requires detectors (FPDs) on at least three layers in total including the front detector, and hence complicates the configuration structure of FPDs and image processing. This can pose problems in terms of, for example, the costs and weights of FPDs.
In consideration of the problems in the prior art described above, the present invention provides an image processing technique that can more accurately estimate scattered rays included in a radiation image with a simpler structure and processing.

SOLUTION TO PROBLEM

An image processing apparatus according to one aspect of the present invention is characterized by comprising:

region specifying means for specifying a region composed of one substance from radiation images corresponding to a plurality of energies and obtained by irradiating an object with radiation;
pixel value estimation means for obtaining an estimated pixel value of the radiation image based on a thickness or density of a substance in the region; and
scattered ray estimation means for estimating a scattered ray included in the radiation image from a difference between a pixel value of the radiation image and the estimated pixel value.

ADVANTAGEOUS EFFECTS OF INVENTION

The present invention makes it possible to more accurately estimate scattered rays included in a radiation image. This can generate a soft tissue image and a bone image separated with higher accuracy by estimating scattered rays in an image obtained in energy subtraction and reducing the estimated scattered rays.
Other features and advantages of the present invention will be apparent from the following description taken in conjunction with the accompanying drawings. Note that the same reference numerals denote the same or like components throughout the accompanying drawings.

BRIEF DESCRIPTION OF DRAWINGS

The accompanying drawings, which are incorporated in and constitute a part of the specification, illustrate embodiments of the invention and, together with the description, serve to explain principles of the invention.

Fig. 1 is a block diagram showing an example of the arrangement of a radiation imaging system according to the first embodiment;
Fig. 2 is a flowchart showing processing by an image processing unit according to the first embodiment;
Fig. 3 shows a view 3a exemplifying a high-energy radiation image, a view 3b exemplifying a low-energy radiation image, a view 3c exemplifying a substance decomposition image of a soft tissue, and a view 3d exemplifying a substance decomposition image of bones;
Fig. 4 is a view showing imaging experiment results according to the first embodiment;
Fig. 5 is a block diagram showing an example of the arrangement of a radiation imaging system according to the second embodiment;
Fig. 6 is a flowchart showing processing by an image processing unit according to the second embodiment; and
Fig. 7 is a view showing imaging experiment results according to the second embodiment.

DESCRIPTION OF EMBODIMENTS

Hereinafter, embodiments will be described in detail with reference to the attached drawings. Note, the following embodiments are not intended to limit the scope of the claimed invention. Multiple features are described in the embodiments, but limitation is not made to an invention that requires all such features, and multiple such features may be combined as appropriate. Furthermore, in the attached drawings, the same reference numerals are given to the same or similar configurations, and redundant description thereof is omitted.

<First Embodiment>

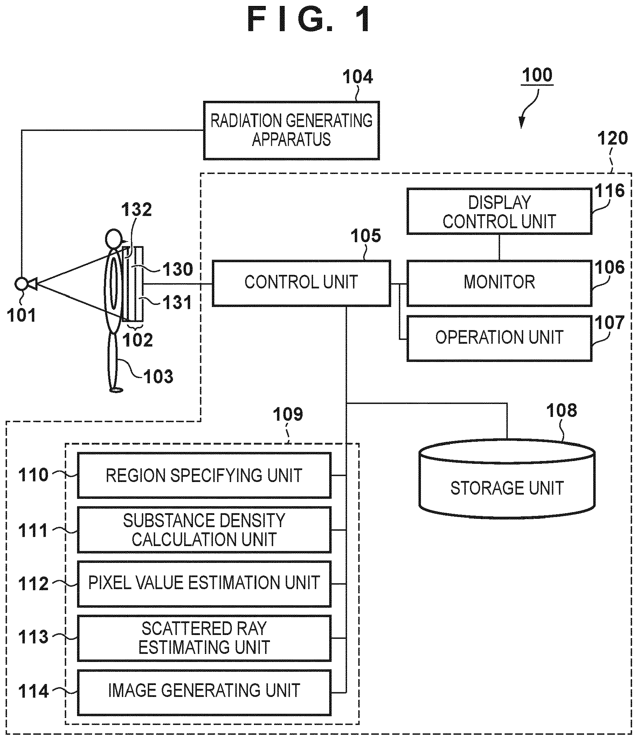

Fig. 1 is a block diagram showing an example of the arrangement of a radiation imaging system 100 according to the first embodiment. The radiation imaging system 100 includes a radiation generating apparatus 104, a radiation tube 101, an FPD 102 (radiation detector), and an information processing apparatus 120. The information processing apparatus 120 processes information based on a radiation image captured by imaging an object. Note that the arrangement of the radiation imaging system 100 is also simply referred to as a radiation imaging apparatus.

The radiation generating apparatus 104 generates radiation by applying high-voltage pulses to the radiation tube 101 by a user operation on the exposure switch (not shown). Note that the term radiation can include, for example, $\alpha$-rays, $\beta$-rays, and $\gamma$-rays, particle rays, and cosmic rays in addition to X-rays. In the first embodiment, although the type of radiation to be used is not specifically limited, X-rays are mainly used for medical image diagnosis. An object 103 is irradiated with radiation generated by the radiation tube 101. Part of the radiation is transmitted through the object 103 and reaches the FPD 102.

The FPD 102 includes a radiation detection unit having a pixel array for generating an image signal corresponding to radiation. The FPD 102 obtains a radiation image by accumulating electric charge based on an image signal and transfers the image to the information processing apparatus 120. The FPD 102 includes a first radiation detection unit 130 and a second radiation detection unit 131 each having a pixel array for generating a signal corresponding to a radiation intensity. The first radiation detection unit 130 and the second radiation detection unit 131 each detect radiation transmitted through the object 103 as an image signal.

The first radiation detection unit 130 and the second radiation detection unit 131 each have an array (two-dimensional region) of pixels that output signals corresponding to incident light. The photoelectric conversion element of each pixel converts radiation converted into visible light by a phosphor into an electrical signal and outputs it as an image signal. The first radiation detection unit 130 and the second radiation detection unit 131 each are configured to obtain an image signal (radiation image) by detecting radiation transmitted through the object 103 in this manner. Of the plurality of radiation detection units, the first radiation detection unit 130 arranged at a position near the radiation tube 101, which generates radiation, generates a low-energy radiation image corresponding to a low energy of a plurality of energies. The second radiation detection unit 131 arranged at a position away from the radiation tube 101 generates a high-energy radiation image corresponding to a high energy compared with a low energy.

The driving unit (not shown) of the FPD 102 outputs an image signal (radiation image) read out in accordance with an instruction from a control unit 105 to the control unit 105. A region specifying unit 110 performs the processing of specifying a region composed of one substance based on radiation images output from the plurality of radiation detection units (the first radiation detection unit 130 and the second radiation detection unit 131) stacked on each other by single irradiation with radiation.

A scattered ray removal grid 132 suppresses scattered rays generated by the object 103 from entering the FPD 102. This embodiment exemplifies the case in which the scattered ray removal grid 132 is arranged between the object 103 and the first radiation detection unit 130. However, this arrangement is not exhaustive, and the scattered ray removal grid 132 may be arranged between the first radiation detection unit 130 and the second radiation detection unit 131.

The information processing apparatus 120 processes information based on a radiation image captured by imaging an object. The information processing apparatus 120 includes the control unit 105, a monitor 106, an operation unit 107, a storage unit 108, an image processing unit 109, and a display control unit 116.

The control unit 105 includes one or a plurality of processors (not shown) and implements various types of control of the information processing apparatus 120 by executing programs stored in the storage unit 108. The storage unit 108 stores image processing results and various types of programs. The storage unit 108 is constituted by, for example, a ROM (Read Only Memory), a RAM (Random Access Memory), and the like. The storage unit 108 can store images output from the control unit 105, images processed by the image processing unit 109, and the calculation results obtained by the image processing unit 109.

The image processing unit 109 processes the radiation image obtained from the FPD 102. The image processing unit 109 includes, as functional components, the region specifying unit 110, a substance density calculation unit 111, a pixel value estimation unit 112, a scattered ray estimation unit 113, and an image generating unit 114. These functional components may be implemented by causing the processor of the control unit 105 to execute predetermined programs or may be implemented by causing one or a plurality of processors of the image processing unit 109 to use programs read from the storage unit 108. The control unit 105, that is, the processor of the image processing unit 109, is formed from, for example, a CPU (Central Processing Unit). The respective components of the image processing unit 109 may be formed from integrated circuits and the like as long as they implement similar functions. The information processing apparatus 120 can be constituted by, as internal components, a graphic control unit such as a GPU (Graphics Processing Unit), a communication unit such as a network card, and input/output control units such as a keyboard and a display or touch panel.

The monitor 106 (display unit) displays the radiation image (digital image) received by the control unit 105 from the FPD 102 and the image processed by the image processing unit 109. The display control unit 116 controls display on the monitor 106 (display unit). The operation unit 107 can input instructions with respect to the image processing unit 109 and the FPD 102, and receives instructions with respect to the FPD 102 via a user interface (not shown).

In the above arrangement, the radiation generating apparatus 104 applies a high voltage to the radiation tube 101 to irradiate the object 103 with radiation. The FPD 102 functions as an obtaining unit to obtain a plurality of radiation images corresponding to a plurality of energies, which are obtained by irradiating the object 103 with radiation. The FPD 102 generates two radiation images with different radiation energies by irradiation with these types of radiation. The radiation images corresponding to the plurality of energies include a low-energy radiation image and a high-energy radiation image generated based on a higher radiation energy than the low-energy radiation image.

In this embodiment, the first radiation detection unit 130 generates a low-energy radiation image $I_L$, and the second radiation detection unit 131 generates a high-energy radiation image $I_H$. In general, the X-rays emitted from the radiation tube 101 has an X-ray spectrum spreading in a wide energy band because the X-rays are generated by bremsstrahlung of an electron beam.

Accordingly, when radiation is transmitted through the first radiation detection unit 130, low-energy components in the X-ray spectrum are absorbed, resulting in beam hardening. As a result, radiation with relatively high energies enters the second radiation detection unit 131.

The region specifying unit 110 specifies a region composed of one substance from radiation images corresponding to a plurality of energies obtained by irradiating an object with radiation. The region specifying unit 110 specifies a portion composed of one substance by using the high-energy radiation image $I_H$ and the low-energy radiation image $I_L$. When the object 103 is a person, the object can be roughly separated into two substances, namely, a soft tissue composed of muscle, fat, organ, and the like and bone. A portion where a bone exists includes a soft tissue covering the bone, whereas a portion where no bone exists is composed of a soft tissue. Accordingly, specifying a region where no bone exists can specify a region composed of a soft tissue substance.

The region specifying unit 110 can use various methods as region extraction methods. For example, the region specifying unit 110 can use at least one of region extraction methods including binarization, region expansion, edge detection, and graph cut. Performing machine learning in advance by using many radiation images of the object 103 as teacher data allows the region specifying unit 110 to specify a region composed of one substance by using a region extraction method based on machine learning on the plurality of radiation images obtained by the FPD 102. When radiation images with two different energies are obtained as in this embodiment, generating in advance a bone image by separating a bone makes it possible to accurately execute the above series of processes of the region extraction method.

The substance density calculation unit 111 calculates the thickness or surface density of a soft tissue in the region specified by the region specifying unit 110 by using the high-energy radiation image $I_H$ and the low-energy radiation image $I_L$. In this case, the surface density is the product of the thickness and the volume density, and hence the thickness and the surface density (to be also simply referred to as the "density" hereinafter) have equivalent meanings. The substance density calculation unit 111 calculates the thickness or density of a substance (soft tissue) by using a radiation image (the low-energy radiation image $I_L$ or the high-energy radiation image $I_H$), of radiation images corresponding to a plurality of energies, which corresponds to one of the energies and the mass attenuation coefficient ($\mu_{LA}$ or $\mu_{HA}$) of the substance (soft tissue) corresponding to one energy. Calculation processing by the substance density calculation unit 111 is based on equations (2) and (3) given below.

The pixel value estimation unit 112 obtains an estimated pixel value of a radiation image based on the thickness or density of a substance (soft tissue) in a region composed of one substance. The pixel value estimation unit 112 calculates estimated pixel values of the high-energy radiation image $I_H$ and the low-energy radiation image $I_L$ based on the thickness or density of the substance (soft tissue) calculated by the substance density calculation unit 111 and the mass attenuation coefficient of the substance (soft tissue). Calculation processing by the pixel value estimation unit 112 is based on, for example, equations (4) and (5) given blow.

The scattered ray estimation unit 113 estimates a scattered ray included in a radiation image from the difference between a pixel value of a radiation image and an estimated pixel value. The scattered ray estimation unit 113 estimates a scattered ray from a pixel value (measured pixel value) of a radiation image and the difference between an estimated pixel value $I_{HE}$ of the high-energy radiation image $I_H$ and an estimated pixel value $I_{LE}$ of the low-energy radiation image $I_L$ calculated by the pixel value estimation unit 112. Estimation processing by the scattered ray estimation unit 113 is based on equations (6) and (7) given below.

The image generating unit 114 generates images (a bone image indicating the density of the bone and a soft tissue image indicating the density of the soft tissue) upon reducing scattered rays from radiation images. Processing by the image generating unit 114 is based on, for example, equations (8) to (11) given below.

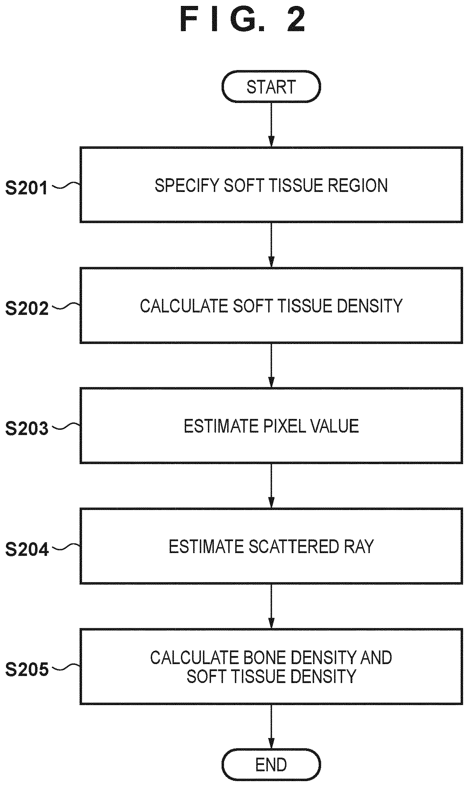

Processing by the image processing unit 109 according to the first embodiment will be described in detail next with reference to the flowchart shown in Fig. 2. The control unit 105 stores the radiation image captured by the FPD 102 in the storage unit 108 and transfers the radiation image to the image processing unit 109.

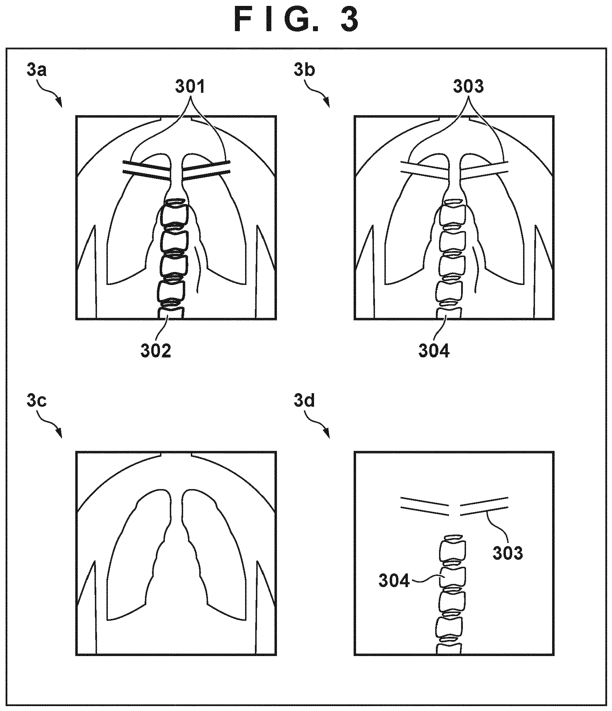

In steps S201 to S205, the image processing unit 109 generates a soft tissue image indicating the density of the soft tissue in 3c of Fig. 3 and a bone image indicating the density of the bone in 3d of Fig. 3, while reducing scattered rays, by using the high-energy radiation image $I_H$ in 3a of Fig. 3 and the low-energy radiation image $I_L$ in 3b of Fig. 3.

In this case, 3a of Fig. 3 is a view exemplifying the high-energy radiation image $I_H$, and 3b of Fig. 3 is a view exemplifying the low-energy radiation image $I_L$. The bone portions (collarbones 303 and vertebrae 304) of the low-energy radiation image $I_L$ in 3b of Fig. 3 are displayed with higher contrast than the bone portions (collarbones 301 and vertebrae 302) of the high-energy radiation image $I_H$ in 3a of Fig. 3.

(Processing by Region Specifying Unit 110)

In step S201, the region specifying unit 110 calculates a bone image $d_B$ in 3d of Fig. 3 by using the high-energy radiation image $I_H$ and the low-energy radiation image $I_L$ according to equation (1) given below.

$$d_B(x,y) = \frac{\mu_{HA}\ln I_L(x,y) - \mu_{LA}\ln I_H(x,y)}{\mu_{HB}\mu_{LA} - \mu_{LB}\mu_{HA}} \qquad \ldots(1)$$

In this case, the bone image $d_B$ indicates a bone density, with the unit being g/cm². In equation (1), the suffixes H and L respectively represent a high energy and a low energy, and the suffices A and B respectively represent substances to be separated (for example, A represents a soft tissue, and B represents a bone). In this case, the soft tissue and the bone are exemplified as substances to be separated. However, they are not exhaustive and arbitrary substances can be used. In this equation, $\mu_{HA}$ is the mass attenuation coefficient of the soft tissue at a high energy, $\mu_{HB}$ is the mass attenuation coefficient of the bone at a high energy, $\mu_{LA}$ is the mass attenuation coefficient of the soft tissue at a low energy, $\mu_{HB}$ is the mass attenuation coefficient of the bone at low energy, x represents a pixel position in the image in the horizontal direction, and y represents a pixel position in the image in the vertical direction.

The bone image $d_B$ in 3d of Fig. 3 has no soft tissue. Accordingly, performing threshold processing makes it possible to specify a region composed of only a soft tissue without any bone like the soft tissue image in 3c of Fig. 3. As threshold processing, for example, the Otsu binarization method as a known technique can be used. Likewise, using a known technique such as a region expansion method, edge detection, or graph cut makes it possible to specify a region composed of only a soft tissue. In addition, when radiation images captured by photographing many objects can be obtained, a region composed of only a soft tissue may be specified by using segmentation based on machine learning.

In this embodiment, the bone image $d_B$ is used to facilitate region extraction. However, a bone region and a region composed of only a soft tissue may be specified from the high-energy radiation image $I_H$ and the low-energy radiation image $I_L$ using a known technique.

(Processing by Substance Density Calculation Unit 111)

In step S202, the substance density calculation unit 111 calculates a soft tissue image $d_A$ in a region composed of only a soft tissue specified in step S201 using the low-energy radiation image $I_L$ according to equation (2) given below.

$$d_A(x, y) = -\frac{\ln I_L(x, y)}{\mu_{LA}} \qquad ...(2)$$

Since it is known that the region specified in step S201 is a region composed of a soft tissue, simple calculation like equation (2) holds. In addition, since the first radiation detection unit 130 is in contact with the scattered ray removal grid 132 in Fig. 1, it is considered that scattered rays have been sufficiently removed. Accordingly, the low-energy radiation image $I_L$ can be approximately considered to suffer little influence of scattered rays. In this embodiment, if the grid ratio of the scattered ray removal grid 132 is represented by the ratio (h/D) of the height (h) of the absorption members (absorption foils) to the interval (D) of the absorption members (absorption foils) constituting the scattered ray removal grid 132, the grid ratio of the scattered ray removal grid 132 can be set to 10 or more. In addition, a cross grid, that is, a grid in a lattice pattern, can be used. The cross grid can reduce scattered rays in the vertical and horizontal directions. This makes it possible to reduce scattered rays isotropically by using the cross grid and increase the scattered ray reducing ability.

As described above, when the scattered ray removal grid 132 is arranged between the first radiation detection unit 130 and the second radiation detection unit 131, the substance density calculation unit 111 can calculate the soft tissue image $d_A$ by using equation (3).

$$d_A(x, y) = -\frac{\ln I_H(x, y)}{\mu_{HA}} \qquad ...(3)$$

(Processing by Pixel Value Estimation Unit 112)

In step S203, the pixel value estimation unit 112 calculates the estimated pixel value $I_{HE}$ of the high-energy radiation image in the soft tissue according to equation (4) given below. The pixel value estimation unit 112 calculates an estimated pixel value of the high-energy radiation image in the substance (soft tissue) based on the soft tissue image $d_A$ (equation (2)) indicating the density of the substance (soft tissue), calculated based on the low-energy radiation image $I_L$, and the mass attenuation coefficient $\mu_{HA}$ of the substance (soft tissue) at the high energy.

$$I_{HE}(x, y) = \mu_{HA} d_A(x, y) \qquad ...(4)$$

In this case, since $d_A(x, y)$ is calculated based on equation (2) from the low-energy radiation image $I_L$ that approximately suffer little influence of scattered rays, the estimated pixel value $I_{HE}(x, y)$ can also be considered to be a high-energy radiation image on which the influence of scattered rays is suppressed.

When the scattered ray removal grid 132 is arranged between the first radiation detection unit 130 and the second radiation detection unit 131, the pixel value estimation unit 112 calculates the estimated pixel value $I_{LE}$ of the low-energy radiation image according to equation (5) given below. The pixel value estimation unit 112 calculates an estimated pixel value of the low-energy radiation image of the substance (soft tissue) based on the soft tissue image $d_A$ (equation (3)) indicating the density of the substance (soft tissue) calculated based on the high-energy radiation image $I_H$ and the mass attenuation coefficient $\mu_{LA}$ of the substance (soft tissue) at the low energy.

$$I_{LE}(x, y) = \mu_{LA} d_A(x, y) \qquad ...(5)$$

(Processing by Scattered Ray Estimation Unit 113)

In step S204, the scattered ray estimation unit 113 calculates a scattered ray $S_H$ of the high-energy radiation image according to equation (6) given below. The scattered ray estimation unit 113 calculates the scattered ray $S_H$ of the high-energy radiation image of the substance (soft tissue) based on the difference between a measured pixel value $I_H(x, y)$ of the high-energy radiation image $I_H$ and the estimated pixel value $I_{HE}(x, y)$ of the high-energy radiation image $I_H$.

$$S_H(x, y) = I_H(x, y) - I_{HE}(x, y) \qquad ...(6)$$

When the scattered ray removal grid 132 is arranged between the first radiation detection unit 130 and the second radiation detection unit 131, a scattered ray $S_L$ of the low-energy radiation image is calculated according to equation (7). That is, the scattered ray estimation unit 113 calculates the scattered ray $S_L$ of the low-energy radiation image of the soft tissue based on the difference between a measured pixel value $I_L(x, y)$ of the low-energy radiation image $I_L$ and an estimated pixel value $I_{LE}(x, y)$ of the low-energy radiation image $I_L$. In this case, the pixel value estimation unit 112 can calculate the estimated pixel value $I_{LE}(x, y)$ of the low-energy radiation image $I_L$ from the product of the mass attenuation coefficient $\mu_{LA}$ of the soft tissue at the low energy and $d_A(x, y)$ in the same manner as indicated by equation (4).

$$S_L(x, y) = I_L(x, y) - I_{LE}(x, y) \qquad ...(7)$$

An estimated scattered ray $S_H(x, y)$ of the high-energy radiation image or an estimated scattered ray $S_L(x, y)$ of the low-energy radiation image is a scattered ray concerning a region composed of only the soft tissue. In the human body, in regions other than regions dominated by bones like the head portion, the areas of the bones are relatively small like the collarbones 303 and the vertebrae 304 in 3d of Fig. 3. Accordingly, the scattered ray estimation unit 113 can estimate scattered rays in a region where the bones exist as a plurality of substances constituting the object by interpolation from a scattered ray estimated pixel value or interpolation based on curved surface fitting in a region composed of only the surrounding substance (soft tissue). In addition, the scattered ray estimation unit 113 can estimate a scattered ray S' in a region where a plurality of substances (bones) constituting the object exist by diffusing scattered ray estimated pixel values in a region composed of only the surrounding substance (soft tissue) using a technique like inpainting processing. In this case, a region composed of only one substance is a region composed of a soft tissue constituting the object, and a region where a plurality of substances exist is a region where the bones constituting the object exist.

(Processing by Image Generating Unit 114)

In step S205, the image generating unit 114 generates images (a bone image and a soft tissue image) obtained by reducing scattered rays from a radiation image. The image generating unit 114 generates an image (soft tissue image) indicating the density of the substance (soft tissue) and an image (bone image) indicating the densities of a plurality of substances (bones) constituting the object by reducing scattered rays from the radiation images by using the scattered rays estimated by the processing (step S204) performed by the scattered ray estimation unit 113.

The image generating unit 114 can calculate a scattered-ray-reduced bone image $d_{BE}(x, y)$ indicating the density of the bone by using a scattered ray $S'_H(x, y)$ of the high-energy radiation image or $S'_L(x, y)$ of the low-energy radiation image based on equation (8) or (9), which is estimated in the bone region. In this case, equation (8) represents the bone image $d_{BE}(x, y)$ which is obtained by reducing the scattered ray $S'_H(x, y)$ of the high-energy radiation image from the high-energy radiation image $I_H(x, y)$ and indicates the density of the bone. Equation (9) represents the bone image $d_{BE}(x, y)$ which is obtained by reducing the scattered ray $S'_L(x, y)$ of the low-energy radiation image from the low-energy radiation image $I_L(x, y)$.

$$d_{BE}(x, y) = \frac{\mu_{HA}\ln I_L(x, y) - \mu_{LA}\ln\{I_H(x, y) - S'_H(x, y)\}}{\mu_{HB}\mu_{LA} - \mu_{LB}\mu_{HA}} \qquad ...(8)$$

$$d_{BE}(x, y) = \frac{\mu_{HA}\ln\{I_L(x, y) - S'_L(x, y)\} - \mu_{LA}\ln I_H(x, y)}{\mu_{HB}\mu_{LA} - \mu_{LB}\mu_{HA}} \qquad ...(9)$$

The image generating unit 114 can calculate a scattered-ray-reduced soft tissue image $d_{BA}(x, y)$ indicating the density of the soft tissue by using the scattered ray $S_H(x, y)$ or $S_L(x, y)$, calculated according to equation (6) or (7), based on equation (10) or (11) given below. Equation (10) represents the soft tissue image $d_{AE}(x, y)$ which is obtained by reducing the scattered ray $S_H(x, y)$ of the high-energy radiation image from the high-energy radiation image $I_H(x, y)$ and indicates the density of the soft tissue. Equation (11) represents the soft tissue image $d_{AE}(x, y)$ which is obtained by reducing the scattered ray $S_L(x, y)$ of the low-energy radiation image from the low-energy radiation image $I_L(x, y)$ and indicates the density of the soft tissue.

$$d_{AE}(x,y) = \frac{\mu_{HB}\ln I_L(x, y) - \mu_{LB}\ln\{I_H(x, y) - S_H(x, y)\}}{\mu_{HB}\mu_{LA} - \mu_{LB}\mu_{HA}} \quad ...(10)$$

$$d_{AE}(x,y) = \frac{\mu_{HA}\ln\{I_L(x, y) - S_L(x, y)\} - \mu_{LA}\ln I_H(x, y)}{\mu_{HB}\mu_{LA} - \mu_{LB}\mu_{HA}} \quad ...(11)$$

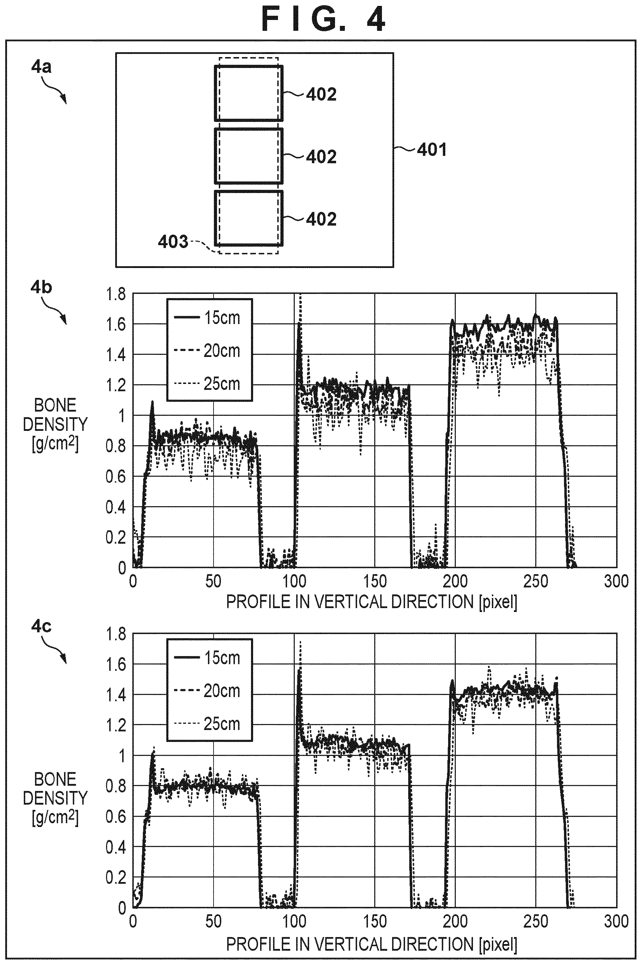

Fig. 4 is a view showing imaging experiment results according to the first embodiment. In Fig. 4, 4a is a view exemplifying the arrangement of a phantom used in the imaging experiments. The phantom mimics the lumbar spine of the human body as the object 103. A region 401 mimicking the soft tissue is formed from polyurethane. Hydroxyapatite is embedded in a region 403 mimicking the lumbar spine.

The scattered ray removal grid 132 used in the imaging experiments is a cross grid having a focal length of 110 cm, a lattice density of 52/cm, and a lattice ratio of 24:1. As shown in Fig. 1, the imaging experiments were executed with the scattered ray removal grid 132 being arranged between the first radiation detection unit 130 and the object 103. The imaging conditions were as follows: a tube voltage of 140 kV, a tube current of 25 mA, and a pulse width of 32 msec. In the imaging experiments, the body thickness dependence of bone images was checked while the thickness of the region 401 (polyurethane) mimicking the soft tissue was changed to 15 cm, 20 cm, and 25 cm.

In Fig. 4, 4b is a view plotting a profile of a region of interest 402 in the vertical direction in a bone image $d_B(x, y)$ indicating the bone density calculated by using equation (1). Referring to 4b of Fig. 4, the solid line indicates a profile when the thickness of the region 401 (polyurethane) is 15 cm, the thick broken line indicates a profile when the thickness is 20 cm, and the thin broken line indicates a profile when the thickness is 25 cm. The bone density profile varied when the body thickness was 15 cm, 20 cm, and 25 cm, and the variation coefficient of the bone density was 6.3%.

According to the standard for approval of X-ray bone density measuring apparatuses (April 1, 2005, Pharmaceutical and Food Safety Bureau Notification No. 0401050), the variation coefficient of body thickness dependence is required to be 2% or less. The variation coefficient (4b of Fig. 4) of bone density calculated by using equation (1) does not meet the standard for approval.

In Fig. 4, 4c is a view plotting a profile of a region of interest 402 in the vertical direction in the bone image $d_E(x, y)$ indicating the bone density calculated by using equation (9) according to the first embodiment. Referring to 4c of Fig. 4, the solid line indicates a profile when the thickness of the region 401 (polyurethane) is 15 cm, the thick broken line indicates a profile when the thickness is 20 cm, and the thin broken line indicates a profile when the thickness is 25 cm. The variations in bone density profile when the body thickness is 15 cm, 20 cm, and 25 cm are suppressed as compared with the case of 4b of Fig. 4. The variation coefficient of the bone density is 1.1%. This indicates that the variation coefficient (4c of Fig. 4) of the bone densities calculated by using equation (9) according to the first embodiment meets the standard for approval described above.

As described above, according to the first embodiment, a soft tissue image and a bone image separated with higher accuracy can be generated by estimating scattered rays in an image obtained by energy subtraction and reducing the estimated scattered rays. In addition, a bone density can be calculated with higher accuracy.

<Second Embodiment>

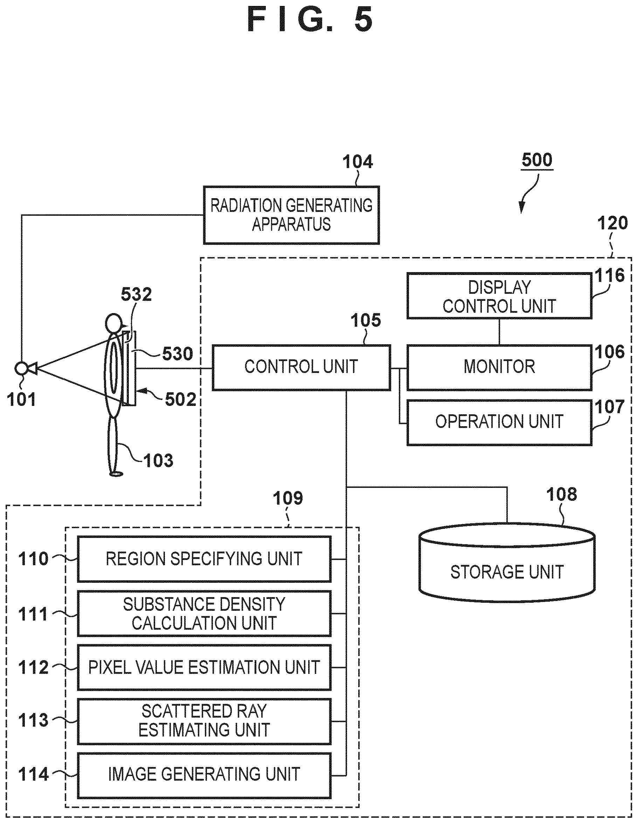

The first embodiment has exemplified the case in which the FPD 102 is formed by stacking the first radiation detection unit 130 and the second radiation detection unit 131. The second embodiment will exemplify a case in which an FPD 502 (radiation detector) includes one radiation detection unit 530, as shown in Fig. 5. Note that the arrangement of a radiation imaging system 500 is the same as that of the radiation imaging system 100 (Fig. 1) described in the first embodiment except for the FPD 502. In the following description, a description of the same part as that of the first embodiment will be omitted, and only part of the arrangement which is unique to the second embodiment will be described.

According to the first embodiment, the first radiation detection unit 130 and the second radiation detection unit 131 respectively obtain the low-energy radiation image $I_L$ and the high-energy radiation image $I_H$ by using the beam hardening effect. However, even when the FPD 502 includes only one radiation detection unit 530 as in the second embodiment, a low-energy radiation image $I_L$ and a high-energy radiation image $I_H$ can be obtained by irradiating an object with radiation twice upon changing the tube voltage of a radiation tube 101. In this case, a region specifying unit 110 performs the processing of specifying a region composed of one substance by irradiating an object with radiation a plurality of times with different tube voltages based on a plurality of radiation images (the low-energy radiation image $I_L$ and the high-energy radiation image $I_H$) output from the one radiation detection unit 530.

In this case, the tube voltage can be changed within an allowable range of the radiation tube 101. In general, energy subtraction is known to increase the substance decomposition performance and the SN of separated images with an increase in energy difference. The dose of radiation that is transmitted through an object and reaches the FPD 502 decreases with a decrease in tube voltage. Accordingly, the second embodiment is advantageous over the first embodiment in that the difference between high and low energies of radiation can be increased, and imaging conditions such as a tube voltage, a tube current, and a pulse width can be individually selected.

The FPD 502 is constituted by only one radiation detection unit 530 and hence has an advantage in suppressing the cost and weight. On the other hand, the second embodiment differs from the first embodiment in that body motion due to the respiration, pulsation, and the like of an object can have some influence because imaging is required to be performed twice.

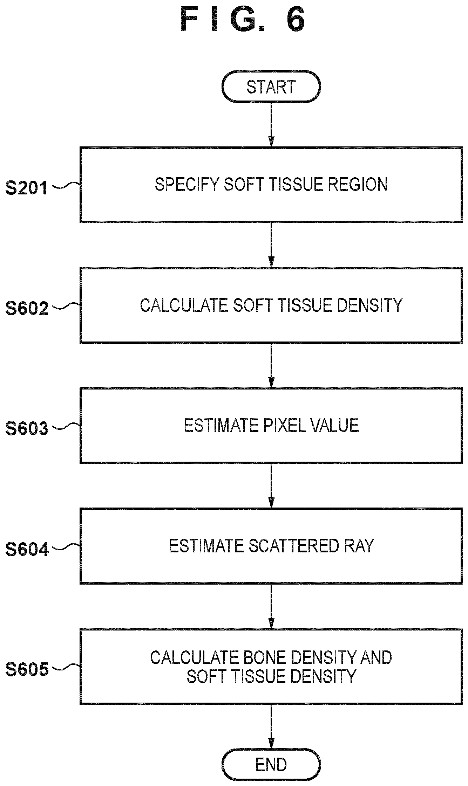

Processing by an image processing unit 109 according to the second embodiment will be described in detail with reference to the flowchart of Fig. 6.

In steps S201 to S605 described below, the image processing unit 109 generates the substance decomposition image (soft tissue image) in 3c of Fig. 3 and the substance decomposition image (bone image) in 3d of Fig. 3, while reducing scattered rays, by using a high-energy radiation image $I_H$ in 3a of Fig. 3 and a low-energy radiation image $I_L$ in 3b of Fig. 3.

(Processing by Region Specifying Unit 110)

In step S201, the region specifying unit 110 specifies a region composed of only a soft tissue (specifying of a soft tissue region). The specifying of the soft tissue region in step S201 is the same as the processing by the region specifying unit 110 described above in the first embodiment.

(Processing by Substance Density Calculation Unit 111)

In step S602, the substance density calculation unit 111 calculates a soft tissue image $d_A$ of the region composed of only the soft tissue specified in step S201 by using the low-energy radiation image $I_L$ according to equation (12).

$$d_A(x, y) = -\frac{\ln I_L(x, y)}{\mu_{LA}} \qquad \ldots(12)$$

Since it is known that this region is composed of only the soft tissue, a simple calculation like equation (12) holds. In the second embodiment, the low-energy radiation image $I_L$ is often captured by imaging at a sufficiently low tube voltage as compared with the high-energy radiation image $I_H$. In general, scattered rays generated by low-energy radiation and caused by an object tend to have large scattering angles, and hence a scattered ray removal grid 532 removes scattered rays more effectively. Accordingly, the low-energy radiation image $I_L$ is considered to be sufficiently subjected to the removal of scattered rays as compared with the high-energy radiation image $I_H$, and hence is approximately considered to suffer little influence of scattered rays.

(Processing by Pixel Value Estimation Unit 112)

In step S603, the pixel value estimation unit 112 calculates the estimated pixel value $I_{HE}$ of the high-energy radiation image of the soft tissue according to equation (13).

$$I_{HE}(x, y) = \mu_{HA} d_A(x, y) \qquad \ldots(13)$$

In this case, $d_A(x, y)$ is calculated from the low-energy radiation image $I_L$ when the influence of scattered rays is approximately small, and hence an estimated pixel value $I_{HE}(x, y)$ can also be considered to be a high-energy radiation image on which the influence of scattered rays is suppressed.

(Processing by Scattered Ray Estimation Unit 113)

In step S604, a scattered ray estimation unit 113 calculates a scattered ray $S_H$ of the high-energy radiation image according to equation (14). The scattered ray estimation unit 113 calculates the scattered ray $S_H$ of the high-energy radiation image of the soft tissue based on the difference between the measured pixel value $I_H(x, y)$ of the high-energy radiation image $I_H$ and the estimated pixel value $I_{HE}(x, y)$ of the high-energy radiation image $I_H$.

$$S_H(x, y) = I_H(x, y) - I_{HE}(x, y)$$

$$...(14)$$

A scattered ray $S'_H(x, y)$ of an energy radiation image estimated in this case is a scattered ray concerning a region composed of only a soft tissue. However, In the human body, in regions other than regions dominated by bones like the head portion, the areas of the bones are relatively small like collarbones 303 and vertebrae 304 in 3d of Fig. 3. Accordingly, scattered rays in a region where the bone exists can be estimated relatively easily by pixel interpolation from a portion composed of only the surrounding soft tissue or interpolation based on curved surface fitting. In addition, a scattered ray in a region where a bone exists can be estimated by diffusing pixels around the bone using a technique like inpainting as a known technique.

(Processing by Image Generating Unit 114)

In step S605, an image generating unit 114 can calculate a bone image $d_{BE}(x, y)$ which is obtained upon reducing scattered rays and indicates the density of the bone based on equation (15) by using a scattered ray $S'_H(x, y)$ of a high-energy radiation image which is estimated in the bone region. In this case, equation (15) is the bone image $d_{BE}(x, y)$ indicating the density of the bone which is obtained by reducing the scattered ray $S'_H(x, y)$ of the high-energy radiation image from the high-energy radiation image $I_H(x, y)$.

$$d_{BE}(x, y) = \frac{\mu_{HA} \ln I_L(x, y) - \mu_{LA} \ln\{I_H(x, y) - S'_H(x, y)\}}{\mu_{HB}\mu_{LA} - \mu_{LB}\mu_{HA}}$$

$$...(15)$$

The image generating unit 114 can also calculate the soft tissue image $d_{AE}(x, y)$ which is obtained upon reducing scattered rays and indicates the density of the soft tissue based on equation (16) using the scattered ray $S_H(x, y)$ calculated by equation (14). Equation (16) is the soft tissue image $d_{AE}(x, y)$ which is obtained upon reducing the scattered ray $S_H(x, y)$ of the high-energy radiation image from the high-energy radiation image $I_H(x, y)$ and indicates the density of the soft tissue.

$$d_{AE}(x, y) = \frac{\mu_{HB} \ln I_L(x, y) - \mu_{LB} \ln\{I_H(x, y) - S_H(x, y)\}}{\mu_{HB}\mu_{LA} - \mu_{LB}\mu_{HA}}$$

$$...(16)$$

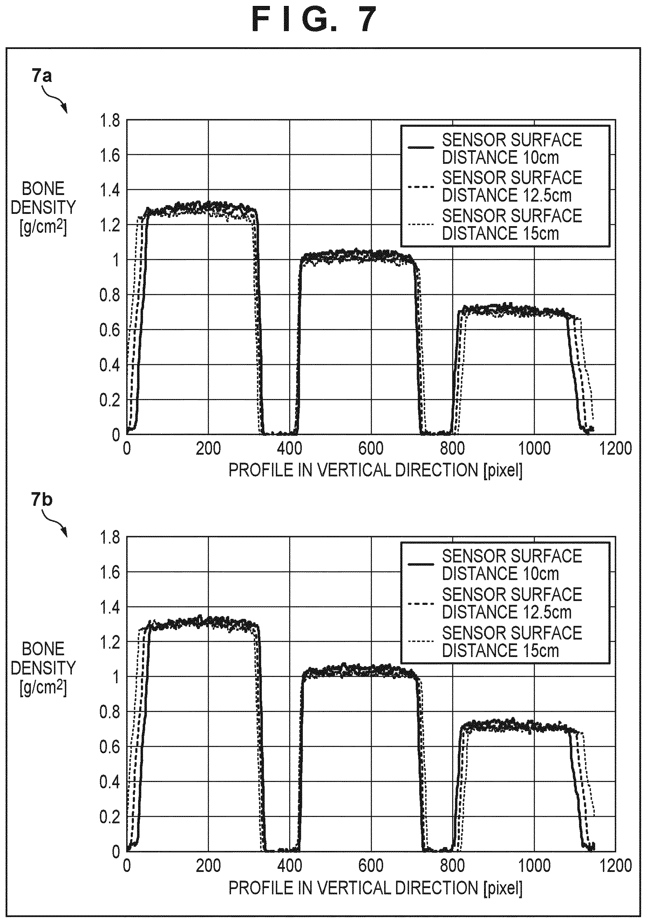

Fig. 7 is a view showing imaging experiment results according to the second embodiment. The arrangement of the phantom used in the imaging experiments is the same as that described with reference to 4a of Fig. 4. The scattered ray removal grid 532 used in the imaging experiments is a cross grid having a focal length of 110 cm, a lattice density of 52/cm, and a lattice ratio of 24:1. As shown in Fig. 5, the imaging experiments were executed with the scattered ray removal grid 532 being arranged between the radiation detection unit 530 and an object 103. The imaging conditions for the high-energy radiation image $I_H$ were as follows: a tube voltage of 140 kV, a tube current of 40 mA, and a pulse width of 32 msec. The imaging conditions for the low-energy radiation image $I_L$ were as follows: a tube voltage of 70 kV, a tube current of 320 mA, and a pulse width of 63 msec.

In the imaging experiments according to this embodiment, the sensor surface distance dependence of bone images indicating bone densities was checked while the distance (to be referred to as the sensor surface distance hereinafter) between the radiation detection unit 530 and the object 103 was changed to 10 cm, 12,5 cm, and 15 cm.

In Fig. 7, 7a is a view plotting a profile of a region of interest 402 in the vertical direction in the bone image $d_B(x, y)$ indicating the bone density calculated by using equation (1). Referring to 7a of Fig. 7, the solid line indicates a profile when the sensor surface distance is 10 cm, the thick broken line indicates a profile when the sensor surface distance is 12.5 cm, and the thin broken line indicates a profile when the sensor surface distance is 15 cm. The bone density profile varied when the sensor surface distance was 10 cm, 12.5 cm, and 15 cm, and the variation coefficient of the bone density was 2.1%.

According to the standard for approval of X-ray bone density measuring apparatuses (April 1, 2005, Pharmaceutical and Food Safety Bureau Notification No. 0401050), the variation coefficient of body thickness dependence is required to be 2% or less. The variation coefficient (7a of Fig. 7) of bone density calculated by using equation (1) does not meet the standard for approval.

In Fig. 7, 7b is a view plotting a profile of the region of interest 402 in the vertical direction in the bone image $d_{BE}(x, y)$ indicating the bone density calculated by using equation (15) according to the second embodiment. Referring to 7b of Fig. 7, the solid line indicates a profile when the sensor surface distance is 10 cm, the thick broken line indicates a profile when the sensor surface distance is 12.5 cm, and the thin broken line indicates a profile when the sensor surface distance is 15 cm. The variations in bone density profile when the sensor surface distance is 10 cm, 12.5 cm, and 15 cm are suppressed as compared with the case of 7a of Fig. 7. The variation coefficient of the bone density is 1.2%. This indicates that the variation coefficient (7b of Fig. 7) of the bone densities calculated by using equation (15) according to the second embodiment meets the standard for approval described above.

As described above, according to the second embodiment, a soft tissue image and a bone image separated with higher accuracy can be generated by estimating scattered rays in an image obtained by energy subtraction and reducing the estimated scattered rays. In addition, a bone density can be calculated with higher accuracy.

(Other Embodiments)

The present invention can be implemented by processing of supplying a program for implementing one or more functions of the above-described embodiments to a system or apparatus via a network or storage medium, and causing one or more processors in the computer of the system or apparatus to read out and execute the program. The present invention can also be implemented by a circuit (for example, an ASIC) for implementing one or more functions.

The present invention is not limited to the above embodiments and various changes and modifications can be made within the spirit and scope of the present invention. Therefore, to apprise the public of the scope of the present invention, the following claims are made.

This application claims the priority on the basis of Japanese Patent Application No. 2019-205686, filed November 13, 2019, which is hereby incorporated by reference herein in its entirety.

REFERENCE SIGNS LIST

100: radiation imaging system, 101: radiation tube, 102: FPD (radiation detector), 104: radiation generating apparatus, 105: control unit, 106: monitor (display unit), 107: operation unit, 108: storage unit, 109: image processing unit, 110: region specifying unit, 111: substance density calculation unit, 112: pixel value estimation unit, 113: scattered ray estimation unit, 114: image generating unit, 120: information processing apparatus, 132: scattered ray removal grid, 502: FPD, 532: scattered ray removal grid

**Claims**

1. An image processing apparatus **characterized by** comprising:

    region specifying means for specifying a region composed of one substance from radiation images corresponding to a plurality of energies and obtained by irradiating an object with radiation;
    pixel value estimation means for obtaining an estimated pixel value of the radiation image based on a thickness or density of a substance in the region; and
    scattered ray estimation means for estimating a scattered ray included in the radiation image from a difference between a pixel value of the radiation image and the estimated pixel value.

2. The image processing apparatus according to claim 1, **characterized by** further comprising image generating means for generating an image obtained by reducing the scattered ray from the radiation image.

3. The image processing apparatus according to claim 1 or 2, **characterized in that** the region specifying means specifies the region by single irradiation with radiation based on the radiation images output from a plurality of radiation detection means stacked on each other.

4. The image processing apparatus according to claim 3, **characterized in that** first radiation detection means, of the plurality of radiation detection means, which is arranged at a position near a radiation tube that generates the radiation generates a low-energy radiation image corresponding to a low energy of the plurality of energies, and second radiation detection means arranged at a position away from the radiation tube generates a high-energy radiation image corresponding to a high energy compared with the low energy.

5. The image processing apparatus according to claim 1 or 2, **characterized in that** the region specifying means specifies the region by a plurality of times of irradiation with radiation with different tube voltages based on the radiation image output from one radiation detection means.

6. The image processing apparatus according to any one of claims 1 to 5, **characterized in that** the region specifying means specifies the region by using at least one region extraction method among a binarization method, a region expansion method, edge detection, graph cut, and a region extraction method based on machine learning with respect to a plurality of radiation images.

7. The image processing apparatus according to any one of claims 1 to 6, **characterized by** further comprising calculation means for calculating a thickness or density of the substance by using a radiation image, of radiation images corresponding to the plurality of energies, which corresponds to one of the energies and a mass attenuation coefficient of the substance corresponding to the one energy.

8. The image processing apparatus according to any one of claims 1 to 7, **characterized in that** the scattered ray estimation means estimates a scattered ray in a region where a plurality of substances constituting the substance exist by at least one process of interpolation of a scattered ray estimated pixel value in the region, interpolation based on curved surface fitting, and inpainting processing.

9. The image processing apparatus according to claim 8, **characterized in that** the radiation images corresponding to the plurality of energies include a low-energy radiation image and a high-energy radiation image generated based on a high radiation energy as compared with the low-energy radiation image.

10. The image processing apparatus according to claim 9, **characterized in that** the pixel value estimation means calculates an estimated pixel value of the radiation image based on a thickness or density of the substance and a mass attenuation coefficient of the substance.

11. The image processing apparatus according to claim 9 or 10, **characterized in that** the pixel value estimation means calculates an estimated pixel value of the high-energy radiation image of the substance based on an image indicating the thickness or density of the substance calculated based on the low-energy radiation image and a mass attenuation coefficient of the substance at a high energy.

12. The image processing apparatus according to claim 11, **characterized in that** the scattered ray estimation means calculates a scattered ray of the high-energy radiation image of the substance based on a difference between a pixel value of the high-energy radiation image and an estimated pixel value of the high-energy radiation image.

13. The image processing apparatus according to claim 9 or 10, **characterized in that** the pixel value estimation means calculates an estimated pixel value of the low-energy radiation image of the substance based on an image indicating the thickness or density of the substance calculated based on the high-energy radiation image and a mass attenuation coefficient of the substance at a low energy.

14. The image processing apparatus according to claim 13, **characterized in that** the scattered ray estimation means calculates a scattered ray of a low-energy radiation image of the substance based on a difference between a pixel value of the low-energy radiation image and an estimated pixel value of the low-energy radiation image.

15. The image processing apparatus according to claim 2, **characterized in that** the image generating means generates an image indicating densities of a plurality of substances constituting the object upon reducing the scattered ray from the radiation image by using the scattered ray.

16. The image processing apparatus according to any one of claims 8 to 15, **characterized in that** the region composed of the one substance is a region composed of a soft tissue constituting the object, and a region where a plurality of substances exist is a region where a bone constituting the object exists.

17. A radiation imaging apparatus **characterized by** comprising an image processing apparatus defined in any one of claims 1 to 16.

18. An image processing method in an image processing apparatus, the method **characterized by** comprising:

a step of causing region specifying means to specify a region composed of one substance from radiation images corresponding to a plurality of energies and obtained by irradiating an object with radiation;
a step of causing pixel value estimation means to obtain an estimated pixel value of the radiation image based on a thickness or density of a substance in the region; and
a step of causing scattered ray estimation means to estimate a scattered ray included in the radiation image from a difference between a pixel value of the radiation image and the estimated pixel value.

19. The image processing method according to claim 18, **characterized by** further comprising causing image generating means to generate an image upon reducing the scattered ray from the radiation image.

20. A program that causes a computer to execute each step of an image processing method defined in claim 18 or 19.

# F I G. 1

RADIATION GENERATING APPARATUS — 104

100

120

DISPLAY CONTROL UNIT — 116

105 CONTROL UNIT

MONITOR — 106

OPERATION UNIT — 107

132
130
131
101
102
103

109
110 REGION SPECIFYING UNIT
111 SUBSTANCE DENSITY CALCULATION UNIT
112 PIXEL VALUE ESTIMATION UNIT
113 SCATTERED RAY ESTIMATING UNIT
114 IMAGE GENERATING UNIT

108 STORAGE UNIT

# FIG. 2

START

S201 — SPECIFY SOFT TISSUE REGION

S202 — CALCULATE SOFT TISSUE DENSITY

S203 — ESTIMATE PIXEL VALUE

S204 — ESTIMATE SCATTERED RAY

S205 — CALCULATE BONE DENSITY AND SOFT TISSUE DENSITY

END

# F I G. 3

# F I G. 4

4a

4b

BONE DENSITY [g/cm²]

PROFILE IN VERTICAL DIRECTION [pixel]

4c

BONE DENSITY [g/cm²]

PROFILE IN VERTICAL DIRECTION [pixel]

# F I G. 5

# F I G. 6

```
                    ┌─────────┐
                    │  START  │
                    └─────────┘
                         │
                         ▼
        ┌────────────────────────────────────┐
  S201  │      SPECIFY SOFT TISSUE REGION     │
        └────────────────────────────────────┘
                         │
                         ▼
        ┌────────────────────────────────────┐
  S602  │     CALCULATE SOFT TISSUE DENSITY   │
        └────────────────────────────────────┘
                         │
                         ▼
        ┌────────────────────────────────────┐
  S603  │        ESTIMATE PIXEL VALUE         │
        └────────────────────────────────────┘
                         │
                         ▼
        ┌────────────────────────────────────┐
  S604  │       ESTIMATE SCATTERED RAY        │
        └────────────────────────────────────┘
                         │
                         ▼
        ┌────────────────────────────────────┐
  S605  │   CALCULATE BONE DENSITY AND        │
        │       SOFT TISSUE DENSITY           │
        └────────────────────────────────────┘
                         │
                         ▼
                    ┌─────────┐
                    │   END   │
                    └─────────┘
```

# F I G. 7

## INTERNATIONAL SEARCH REPORT

| International application No. |
| --- |
| PCT/JP2020/039232 |

A. CLASSIFICATION OF SUBJECT MATTER
Int. Cl. A61B6/00(2006.01)i
FI: A61B6/00 333, A61B6/00 350S

According to International Patent Classification (IPC) or to both national classification and IPC

B. FIELDS SEARCHED

Minimum documentation searched (classification system followed by classification symbols)
Int. Cl. A61B6/00-6/14

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2002-171444 A (FUJI PHOTO FILM CO., LTD.) 14 June 2002, entire text, all drawings | 1-20 |
| A | JP 2015-181649 A (FUJIFILM CORP.) 22 October 2015, entire text, all drawings | 1-20 |
| A | JP 2018-511443 A (CASE WESTERN RESERVE UNIVERSITY) 26 April 2018, paragraphs [0253]-[0255] | 1-20 |
| A | JP 10-118056 A (FUJI PHOTO FILM CO., LTD.) 12 May 1998, entire text, all drawings | 1-20 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 30.11.2020 | 15.12.2020 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

Information on patent family members

| | | International application No. |
|---|---|---|
| | | PCT/JP2020/039232 |

| Patent Documents referred to in the Report | Publication Date | Patent Family | Publication Date |
|---|---|---|---|
| JP 2002-171444 A | 14.06.2002 | (Family: none) | |
| JP 2015-181649 A | 22.10.2015 | US 2017/0055933 A1 entire text, all drawings WO 2015/146011 A1 | |
| JP 2018-511443 A | 26.04.2018 | WO 2016/168194 A1 paragraphs [0118]-[0120] US 2018/0042565 A1 CA 2982526 A1 | |
| JP 10-118056 A | 12.05.1998 | US 6075877 A entire text, all drawings | |

Form PCT/ISA/210 (patent family annex) (January 2015)

22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3476644 B
- JP 2019205686 A